(19)

Europäisches
Patentamt
European
Patent Office
Office européen
des brevets

(11) **EP 4 226 847 A1**

(12) **EUROPEAN PATENT APPLICATION**

(43) Date of publication:
**16.08.2023 Bulletin 2023/33**

(21) Application number: **22156860.3**

(22) Date of filing: **15.02.2022**

(51) International Patent Classification (IPC):
**A61B 5/00** (2006.01)　　　**A61B 5/08** (2006.01)

(52) Cooperative Patent Classification (CPC):
**A61B 5/4088; A61B 5/082**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Otto-von-Guericke-Universität
Magdeburg
39106 Magdeburg (DE)**

(72) Inventors:
- **PROF. FRODL, Thomas
  39108 Magdeburg (DE)**
- **MEYER-LOTZ, Gabriela
  39116 Magdeburg (DE)**
- **DOBROWOLNY, Henrik
  39175 Gerwisch (DE)**
- **PROF. HOESCHEN, Christoph
  39114 Magdeburg (DE)**

(54) **NOVEL BIOMARKERS FOR DIAGNOSING SCHIZOPHRENIA IN EXHALED BREATH**

(57)　The present invention relates to a method of diagnosing schizophrenia in a patient. Further, the present invention relates to a method of determining the course of schizophrenia in a patient. Furthermore, the present invention relates to a method of determining whether a patient responds to a therapeutic treatment of schizophrenia. In addition, the present invention relates to a device which can be used in these methods. Moreover, the present invention relates to a kit comprising the device.

**FIGURE 1**

| variable | Schizophrenia (n=28) | Control (n=29) | Test | test value | p-value | effect size test | effect size | effect size interpretation |
|---|---|---|---|---|---|---|---|---|
| Age [years] | 39.07 ± 10.27 | 33.86 ± 7.87 | T | T = 2.14 | 0.037* | Cohen's | 0.571 | middle |
| Gender | f: 15 / m: 13 | f: 17 / m: 12 | X² | X²=0.014 | 0.907 | Cramers | 0.051 | small |
| School [years] | 11.2 ± 1.15 | 10.4 ± 1.26 | T | T = 2.44 | 0.018* | | | |
| BMI [kg/m²] | 28.20 ± 5.47 | 24.56 ± 3.68 | T | T = 2.94 | 0.005** | Cohen's | 0.783 | middle |
| Smoking [n] | Yes: 16 / No: 12 | Yes: 2 / No: 27 | X² | X²=14.40 | <0.001*** | Cramers | 0.540 | middle |
| Alcohol use [n] | Yes: 4 / No: 24 | Yes: 6 / No: 23 | X² | X²=0.082 | 0.774 | Cramers | 0.084 | small |
| First episode / exazerbation [n] | 3/25 | | | | | | | |
| Total Illness duration [years] | 12.0 ± 9.8 | | | | | | | |
| PANSS Score<br>PANSS positive<br>PANSS negative | 51.6 ± 10.1<br>11.7 ± 3.3<br>13.1 ± 3.7 | - | - | - | | - | - | - |
| BDI-II | 20.00 ± 12.38 | 2.069 ± 3.807 | T | T = 7.21 | <0.001*** | Cohen's | 1.988 | large |
| Olanzapine equivalents [mg/day] | 21.36 ± 13.83 | | | | | | | |
| None/Monotherapy/Combination [1] | 1/12/15 | | | | | | | |
| Receptor profile, None / risperidone / olanzapine / partial agonists / combination | 1/7/6/2/12 | | | | | | | |
| Days in Hospital [days] | 42.0 ± 35.1 | | | | | | | |

[1] At time of measurement 1 patient did not take antipsychotics, 12 were taking a monotherapy of second generation antipsychotics (4 risperidone, 3 olanzapine, 2 quetiapine, 1 clozapine, 1 cariprazine, 1 aripiprazole) and 15 a combination therapy (1 clozapine + risperidone, 2 clozapine + haloperidole, 2 olanzapine + aripiprazole, 1 olanzapine + cariprazine, 1 clozapine + paliperidone, 2 paliperidone LAT + risperidone, 1 risperidone + cariprazine, 1 paliperidone LAT + cariprazine, 1 paliperidone LAT + risperidone + olanzapine, 1 aripiprazole + quetiapine, 1 : olanzapine + haloperidol, 1 paliperidone + haloperidol). Five patients additionally received valproate.

EP 4 226 847 A1

**Description**

The present invention relates to a method of diagnosing schizophrenia in a patient. Further, the present invention relates to a method of determining the course of schizophrenia in a patient. Furthermore, the present invention relates to a method of determining whether a patient responds to a therapeutic treatment of schizophrenia. In addition, the present invention relates to a device which can be used in these methods. Moreover, the present invention relates to a kit comprising the device.

BACKGROUND OF THE INVENTION

Schizophrenia, one of the most severe mental disorders, can lead to severe impairments in social, psychological, physical, and professional functioning of a person. Its impact worldwide is further highlighted by the lifetime morbid risk for schizophrenia of 7.2 per 1000 persons. The global age-standardized point prevalence of schizophrenia amounts to 0.28% according to one epidemiological study, which also saw the rise of prevalent cases from 13.1 million to 20.9 million cases globally in 2016.

A particular challenging problem faced by clinicians is the lack of objective markers to support the diagnostic process. Instead, diagnosis is made clinically on the bias of history and by examination of mental state. While the clinical diagnosis will remain the gold standard, additional objective markers could facilitate the recognition of the disorder in general practice and might facilitate the diagnostic process. This is of high importance since a longer duration of untreated psychosis has been linked to poorer short-term and long-term outcome and since early treatment entry might potentially reduce hospitalizations and increase independent living by 41.5-46% as well as rising employment by 42-58%.

Identification of objective biological markers to develop diagnostic tools that aid clinicians is one goal for psychiatry in the already advanced era of precision medicine. There are a few studies in this direction, however none of the detected markers has been translated into clinical practice yet. The known markers are either invasive blood markers or imaging markers that are time consuming, laboratory extensive and delicate, or stressful to obtain. Thus, new non-invasive biomarkers indicative for schizophrenia are highly needed.

The present inventors have identified new non-invasive biomarkers indicative for schizophrenia by breath gas analysis. Breath gas analysis allows measuring the volatile organic compounds (VOCs) found in breathing air of patients, e.g. by use of Tedlar bags that trap the exhaled breath of the patient or by direct measurement. In particular, the present inventors found, by comparing the breath gas of patients suffering from schizophrenia and healthy control subjects, that the exhaled breath gas includes volatile organic compounds (VOCs) that are exhaled shortly after their production and are indicative for the presence of schizophrenia. The advantage of breath gas analysis, e.g. via Proton-Transfer-Reaction Mass Spectrometry (PTR-MS), is that it is non-invasive and can be carried out without many preparations and repeatedly during the time of disease course or even during the same day. As the diagnostic process includes early symptom development as well as the clinical course of disease, breath gas analysis is an easy-to-applicate diagnostic method. It can capture the complete dynamics of the disease, predictive symptoms and the effect of interventions, and can much better represent a disease-specific pattern than a marker that is assessed only once.

Thus, the present inventors have discovered a new, simple, quick, and non-invasive method in the diagnostics of schizophrenia. The breath biomarkers for schizophrenia identified by the present inventors are non-invasive and easy to determine. They allow the diagnosis of schizophrenia. They can also be used to predict short-term disease changes. Using these breath biomarkers, effects of therapeutic treatment can further be examined and, thus, therapy success can be monitored.

SUMMARY OF THE INVENTION

In a first aspect, the present invention relates to a method of diagnosing schizophrenia in a patient (suspected of having schizophrenia) comprising the step of:

    determining the level of at least one compound in a sample of exhaled breath obtained from a patient (suspected of having schizophrenia),
    wherein the at least one compound is selected from the group consisting of trimethylamine (TMA), a substituted hexane, an ester of butyric acid, acetone, isoprene, dihydropyrrole, and methylguanidine.

In a second aspect, the present invention relates to a method of determining the course of schizophrenia in a patient (having schizophrenia) comprising the step of:

    determining the level of at least one compound in a sample of exhaled breath obtained from a patient (having schizophrenia),

wherein the at least one compound is selected from the group consisting of trimethylamine (TMA), a substituted hexane, an ester of butyric acid, acetone, isoprene, dihydropyrrole, and methylguanidine.

In a third aspect, the present invention relates a method of determining whether a patient (having schizophrenia) responds to a therapeutic treatment of schizophrenia comprising the step of:

determining the level of at least one compound in a sample of exhaled breath obtained from a patient (having schizophrenia),
wherein the at least one compound is selected from the group consisting of trimethylamine (TMA), a substituted hexane, an ester of butyric acid, acetone, isoprene, dihydropyrrole, and methylguanidine.

In a fourth aspect, the present invention relates to the use of at least one compound for diagnosing schizophrenia in a patient (suspected of having schizophrenia), for determining the course of schizophrenia in a patient (having schizophrenia), or for determining whether a patient (having schizophrenia) responds to a therapeutic treatment of schizophrenia, wherein the at least one compound is selected from the group consisting of trimethylamine (TMA), a substituted hexane, an ester of butyric acid, acetone, isoprene, dihydropyrrole, and methylguanidine.

In a fifth aspect, the present invention relates to a device comprising

(i) an inlet for receiving exhaled breath, and
(ii) an analysis unit configured to analyze the exhaled breath,

wherein the analysis comprises the determination of the level of at least one compound in a sample of exhaled breath obtained from a patient,
wherein the at least one compound is selected from the group consisting of trimethylamine (TMA), a substituted hexane, an ester of butyric acid, acetone, isoprene, dihydropyrrole, and methylguanidine.

In a sixth aspect, the present invention relates to a kit comprising the device according to the fifth aspect.

This summary of the invention does not necessarily describe all features of the present invention. Other embodiments will become apparent from a review of the ensuing detailed description.

DETAILED DESCRIPTION OF THE INVENTION

Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The term "schizophrenia", as used herein, refers to a mental disorder. In particular, schizophrenia is characterized by continuous or relapsing episodes of psychosis. Major symptoms include hallucinations (typically hearing voices), delusions, paranoia, and disorganized thinking. Other symptoms include social withdrawal, decreased emotional expression, and apathy. Symptoms typically come on gradually, begin in young adulthood, and in many cases never resolve.

A particular challenging problem faced by clinicians is the lack of objective markers to support the diagnostic process. Presently, there is no objective diagnostic test. Instead, diagnosis is made clinically on the bias of history and by examination of mental state. In particular, the diagnosis is used to describe observed behavior that may stem from numerous different causes. Besides observed behavior, doctors will also take a history that includes the patient's reported experiences, and reports of others familiar with the person, when making a diagnosis. To diagnose someone with schizophrenia, doctors are supposed to confirm that symptoms and functional impairment are present for six months (Diagnostic and Statistical Manual of Mental Disorders-5 (DSM-5)) or one month (International Classification of Diseases-11 (ICD-11)). Many people with schizophrenia have other mental disorders, especially substance use disorders, depressive disorders, anxiety disorders, and/or obsessive-compulsive disorders. Schizophrenia can be divided into different subtypes, namely catatonic schizophrenia, disorganized schizophrenia, paranoid schizophrenia, residual schizophrenia, or undifferentiated schizophrenia. In the Diagnostic and Statistical Manual of Mental Disorders-5 (DSM-5), schizophrenia is, however, designated as spectrum disorder that includes these subtypes. Thus, the terms "schizophrenia" and "schizophrenia spectrum disorder" are interchangeably used herein.

While the clinical diagnosis will likely remain the gold standard, additional objective markers could facilitate the recognition of the disorder in general practice and might facilitate the diagnostic process. This is of high importance since a longer duration of untreated psychosis has been linked to poorer short-term and long-term outcome and since early treatment entry might potentially reduce hospitalizations and increase independent living by 41.5-46% as well as rising employment by 42-58%.

The present inventors have identified compounds which allow the non-invasive and fast diagnosis of schizophrenia. Said markers are selected from the group consisting of trimethylamine (TMA), a substituted hexane, an ester of butyric acid, acetone, isoprene, dihydropyrrole, and methylguanidine.

The term "diagnosing schizophrenia", as used herein, means determining whether a patient shows signs of or suffers from schizophrenia.

The term "determining the course of schizophrenia", as used herein, means determining the development of schizophrenia over time, e.g. whether schizophrenia worsens in the patient, does not worsen/is stable/not progressing in the patient, or improves in the patient over time.

The term "determining whether a patient responds to a therapeutic treatment of schizophrenia", as used herein, means determining whether a therapeutic treatment is effective or not, e.g. whether the dose of a drug has to be increased or decreased or whether the drug has to be changed.

The term "Diagnostic and Statistical Manual of Mental Disorders-5 (DSM-5)", as used herein, refers to the fifth update to the Diagnostic and Statistical Manual of Mental Disorders, the taxonomic and diagnostic tool published by the American Psychiatric Association (APA). In the United States, the DSM serves as the principal authority for psychiatric diagnoses. Treatment recommendations, as well as payment by health care providers, are often determined by DSM classifications, so the appearance of a new version has significant practical importance.

The term "patient", as used herein, refers to any subject for whom it is desired to know whether she or he suffers from schizophrenia. In particular, the term "patient", as used herein, refers to a subject suspected to be affected by schizophrenia. The patient may be diagnosed to be affected by schizophrenia, i.e. diseased, or may be diagnosed to be not affected by schizophrenia, i.e. healthy. The term "patient", as used herein, also refers to a subject which is affected by schizophrenia, i.e. diseased. The patient may be retested for schizophrenia and may be diagnosed to be still affected by schizophrenia, i.e. diseased, or not affected by schizophrenia anymore, i.e. healthy, for example after therapeutic intervention. The patient may also be retested for schizophrenia and may be diagnosed as having developed an advanced form of schizophrenia.

It should be noted that a patient that is diagnosed as being healthy, i.e. not suffering from schizophrenia, may possibly suffer from another disease not tested/known.

The patient may be any mammal, including both a human and another mammal, e.g. an animal. Human patients are particularly preferred.

The term "(control) subject", as used herein, refers to a subject known to be affected by schizophrenia (positive control), i.e. diseased. Said (control) subject may have developed an advanced form of schizophrenia. The term "(control) subject", as used herein, also refers to a subject known to be not affected by schizophrenia (negative control), i.e. healthy. Thus, the term "healthy subject", as used herein, means a subject which is known to be not affected schizophrenia.

It should be noted that a (control) subject which is known to be healthy, i.e. not suffering from schizophrenia, may possibly suffer from another disease not tested/known.

The (control) subject may be any mammal, including both a human and another mammal, e.g. an animal.

Human (control) subjects are particularly preferred.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease.

The treatment of schizophrenia includes, but is not limited to, the administration of a drug, psychotherapy, exercise training, cognitive remediation, cognitive training, and/or physical rehabilitation.

Preferably, the drug to be used in said treatment is selected from the group consisting of an antipsychotic drug, an atypical antipsychotic drug, and a typical antipsychotic drug.

More preferably, the antipsychotic drug is selected from the group consisting of clozapine, risperidone, olanzapine, aripiprazole, lumateperone, quetiapine, paliperidone, amisulpride, cariprazine, asenapine, loxapine, lurasidone, ziprasidone, sertindol, brexpiprazol, benperidol, bromperidol, chlorprotixen, flupentixol, fluphenazin, fluspirilen, haloperidol, levomepromazine, melperon, perazin, perphenazin, pimozid, pipamperone, prothipendyl, sulpiride, thioridazine, and zuclopenthixol.

The term "breathomics", as used herein, refers to the study of compounds found in exhaled air. The field of breathomics mainly focuses on health-related volatile organic compounds (VOCs). Since the amount of these compounds varies with health status, breathomics holds great promise to deliver non-invasive diagnostic tools. Thus, the main aim of breathomics is to find patterns of VOCs related to abnormal metabolic processes occurring in the human or animal body. The origin of breath VOCs include the environment (termed exogenous), the host (endogenous), and also the microbiome (the microorganisms that inhabit the mouth, lung and gut). Exhaled breath is predominantly composed of nitrogen, oxygen, carbon dioxide, argon as well as water vapour, whereas the volatile organic compounds (VOCs) which may be diagnostically useful are only found in low concentrations. However, modern analytical instruments made the identification of VOCs patterns via human or animal olfaction for disease diagnosis more reliable and robust. The advantage of breath diagnosis is its non-invasive nature, with the ability to take repeat measurements with little stress or discomfort to the individual under investigation.

The present inventors surprisingly identified compounds which are deregulated between patients suffering from schizophrenia and healthy subjects. Said compounds are selected from the group consisting of trimethylamine (TMA), a substituted hexane, an ester of butyric acid, acetone, isoprene, dihydropyrrole, and methylguanidine.

The compounds identified by the present inventors can also be designated as volatile organic compounds (VOCs).

The term "sample of exhaled breath", as used herein, refers to gas sample derived from the body of a patient or (control) subject containing the at least one compound selected from the group consisting of trimethylamine (TMA), a substituted hexane, an ester of butyric acid, acetone, isoprene, dihydropyrrole, and methylguanidine.

The gas sample derived from the body of a patient or (control) subject encompasses exhaled condensate and exhaled gas. If the gas sample is obtained from patients to be tested, it is simply designated as "sample of exhaled breath". If the gas sample is obtained from control subjects, it is designated as "reference sample of exhaled breath".

The term "sensitivity", as used herein, refers to the number of true positive patients (%) with regard to the number of all patients (100%). The patients suffer from schizophrenia. The sensitivity is calculated by the following formula: Sensitivity= TP/(TP+FN) (TP= true positives; FN=false negatives).

The term "specificity", as used herein, relates to the number of true negative individuals (%) with regard to the number of all healthy subjects (100%). The specificity is calculated by the following formula: Specificity= TN/(TN+FP) (TN= true negatives; FP=false positives).

The term "accuracy", as used herein, means a statistical measure for the correctness of classification or identification of sample types. The accuracy is the proportion of true results (both true positives and true negatives).

The term "AUC", as used herein, relates to an abbreviation for the area under a curve. In particular, it refers to the area under a Receiver Operating Characteristic (ROC) curve. The term "Receiver Operating Characteristic (ROC) curve", as used herein, refers to a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve is a measure for the accuracy of a diagnostic test (the larger the area the better, optimum is 1, a random test would have a ROC curve lying on the diagonal with an area of 0.5 (see, for reference, for example, JP. Egan. Signal Detection Theory and ROC Analysis).

The term "level", as used herein, refers to an amount (measured for example in grams, mole, or ion counts) or concentration (e.g. absolute or relative concentration) of the at least one compound selected from the group consisting of trimethylamine (TMA), a substituted hexane, an ester of butyric acid, acetone, isoprene, dihydropyrrole, and methylguanidine.

The term "level", as used herein, also comprises scaled, normalized, or scaled and normalized amounts or values. The level may also be a cut-off level. The level may further be an average level. It may be determined by measuring levels and calculating the "average" amount or value (e.g. mean, median or modal amount or value) thereof.

If the level is determined in an exhaled breath sample obtained from patients which are to be tested, it is simply designated as "level". If the level is determined in an exhaled breath sample obtained from control subjects, it is designated as "reference level".

The level of the at least one compound is preferably determined by spectrometry, chromatography, or a combination of both. The spectrometry is specifically mass spectrometry (MS), and/or the chromatography is specifically gas chromatography (GC), liquid chromatography (LC) or affinity chromatography. Especially, the mass spectrometry (MS) is selected from the group consisting of Proton-Transfer-Reaction mass spectrometry (PTR-MS), Time of Flight mass spectrometry (TOF-MS), and quadrupole mass spectrometry (QMS).

The term "mass spectrometry (MS)", as used herein, refers to the use of an ionization source to generate gas phase ions from a sample on a surface and detecting the gas phase ions with a mass spectrometer. The mass spectrometry is preferably selected from the group consisting of proton-transfer-reaction mass spectrometry (PTR-MS), time of flight mass spectrometry (TOF-MS), and quadrupole mass spectrometry (QMS).

The general concept of PTR-MS is to bring the breath gas into a drift tube. For this purpose, the gas may be mixed and thus react with protons ($H_3O^+$ - ions). The protonated VOCs will then be analyzed by the mass spectrometry part. Typically, masses between m/z 19 and 200 can be analyzed with the quadrupole based analyzer unit and masses between m/z 19 and about 800 can be analyzed with the TOF based analyzer. The linear range of the system is between a few volume parts per trillion (pptv) and a few hundred parts per billion (ppbv).

The term "liquid chromatography (LC)", as used herein, refers to a separation technique in which the mobile phase is a liquid. It can be carried out either in a column or a plane. A liquid chromatography that generally utilizes very small packing particles and a relatively high pressure is referred to as high performance liquid chromatography (HPLC). In HPLC, the sample is forced by a liquid at high pressure (the mobile phase) through a column that is packed with a stationary phase composed of irregularly or spherically shaped particles, a porous monolithic layer, or a porous membrane.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of patient care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the patient. This increases the likelihood that the patient, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the patient so that the treatment plan can be adjusted as necessary before the patient leaves.

Embodiments of the invention

The present inventors have chosen the breath gas analysis in order to identify new non-invasive biomarkers indicative for schizophrenia. By comparing the breath gas of patients suffering from schizophrenia and healthy control subjects, the present inventors surprisingly found that the exhaled breath gas includes volatile organic compounds (VOCs) that are exhaled shortly after their production and are indicative for the presence of schizophrenia. Because the lungs act as an exchange system for gas between the internal system and external environment, the internal system in disorders like schizophrenia can be assessed through the analysis of the volatile organic compounds in the exhaled breath. These breath biomarkers are non-invasive and easy to determine. They allow the diagnosis of schizophrenia. These breath biomarkers can also be used to predict short-term disease changes. Using these breath biomarkers, effects of therapeutic treatment can further be examined and, thus, therapy success can be monitored.

Thus, in a first aspect, the present invention relates to a method of diagnosing schizophrenia in a patient (suspected of having schizophrenia) comprising the step of: determining the level of at least one compound (e.g. at least 1, 2, 3, 4, 5, 6, or 7 compound(s)) in a sample of exhaled breath obtained from a patient (suspected of having schizophrenia), wherein the at least one compound (e.g. at least 1, 2, 3, 4, 5, 6, or 7 compound(s)) is selected from the group consisting of trimethylamine (TMA), a substituted hexane, an ester of butyric acid, acetone, isoprene, dihydropyrrole, and methylguanidine.

Preferably, the at least one compound is selected from the group consisting of trimethylamine (TMA), a substituted hexane, more preferably 1-hexanol or 2-hexanole, and an ester of butyric acid.

For example, the level of trimethylamine (TMA), the level of a substituted hexane, or the level of an ester of butyric acid is determined. For example, the level of trimethylamine (TMA) is determined and the level of a substituted hexane is determined. For example, the level of trimethylamine (TMA) is determined and the level of an ester of butyric acid is determined. For example, the level of a substituted hexane is determined and the level of an ester of butyric acid is determined. For example, the level of trimethylamine (TMA) is determined, the level of a substituted hexane is determined, and the level of an ester of butyric acid is determined. The substituted hexane is specifically 1-hexanol or 2-hexanole.

In one embodiment, the level of the at least one compound is compared to a reference level of said at least one compound. Thus, in one particular embodiment, the present invention relates to a method of diagnosing schizophrenia in a patient (suspected of having schizophrenia) which comprises the steps of:

(i) determining the level of at least one compound (e.g. at least 1, 2, 3, 4, 5, 6, or 7 compound(s)) in a sample of exhaled breath obtained from a patient (suspected of having schizophrenia), and
(ii) comparing the level of the at least one compound (e.g. at least 1, 2, 3, 4, 5, 6, or 7 compound(s)) to a reference level of said at least one compound,

wherein the at least one compound is selected from the group consisting of trimethylamine (TMA), a substituted hexane, an ester of butyric acid, acetone, isoprene, dihydropyrrole, and methylguanidine.

The above comparison allows to diagnose schizophrenia in the patient suspected of having schizophrenia. The patient may be diagnosed as suffering from schizophrenia, i.e. being diseased, or as not suffering from schizophrenia, i.e. being healthy.

The reference level may be any level which allows to determine whether a patient suffers from schizophrenia or not. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one healthy subject, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference samples of exhaled breath obtained from between 2 and 500 healthy subjects. It is even more preferred that the reference level is determined by measuring between 50 and 500 reference samples of exhaled breath obtained from between 50 and 500 healthy subjects. It is most preferred that the reference level is determined by measuring between 100 and 500 reference samples of exhaled breath obtained from between 100 and 500 healthy subjects.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

As mentioned above, the level of the at least one compound is compared to a reference level of said at least one compound. Said reference level is the level determined by measuring a reference sample of exhaled breath. For example, if the level of trimethylamine (TMA) is determined in a sample of exhaled breath obtained from a patient, it is compared to a reference level of trimethylamine (TMA) determined in a reference sample of exhaled breath. Alternatively, if the level of trimethylamine (TMA) and the level of isoprene are determined in a sample of exhaled breath obtained from a patient, both levels are compared to the respective reference levels, i.e. the level of trimethylamine (TMA) is compared to the reference level of trimethylamine (TMA) and the level of isoprene is compared to the reference level of isoprene determined in a reference sample of exhaled breath.

It is further (additionally or alternatively) preferred that the level of the at least one compound below the reference level indicates that the patient has schizophrenia.

In particular, the level of the at least one compound is at least 0.5-fold, more preferably at least 1-fold, even more preferably at least 1.5-fold, still more preferably at least 2-fold, and most preferably at least 3-fold below the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below the reference level.

In principle, the determination of the level of the at least one compound can take place at any time of day. The present inventors found that the levels of the at least one compound are stable over time, in particular also over the time of awakening, to that the determination can take place indeed at any time of the day. However, the present inventors found that the time after awakening/getting up is particularly suitable for breath gas analysis. Thus, it is also (additionally or alternatively) preferred that the level of the at least one compound is determined in a sample of exhaled breath obtained from a patient (suspected of having schizophrenia) at a point in time within 0 and 5 minutes after awakening (= at awakening or immediately after awakening), at a point in time within < 5 minutes and 30 minutes after awakening, or at a point in time within < 30 minutes and 60 minutes after awakening.

In particular, the level of the at least one compound is determined in a sample of exhaled breath obtained from a patient (suspected of having schizophrenia) about 2 minutes ($\pm$ 1 minute) after awakening, 30 minutes after awakening, or 60 minutes after awakening.

It is more preferred that the level of the at least one compound represents an average level of levels determined in a sample of exhaled breath obtained from a patient (suspected of having schizophrenia) at a point in time within 0 and 5 minutes after awakening (= at awakening or immediately after awakening), at a point in time within < 5 minutes and 30 minutes after awakening, and at a point in time within < 30 minutes and 60 minutes after awakening (= baseline level).

For example, the level of the at least one compound (e.g. isoprene) is determined in a sample of exhaled breath obtained from a patient at a point in time within 0 and 5 minutes after awakening, the level of the (same) at least one compound (e.g. isoprene) is determined in a sample of exhaled breath obtained from the (same) patient at a point in time within < 5 minutes and 30 minutes after awakening, and the level of the (same) at least one compound (e.g. isoprene) is determined in a sample of exhaled breath obtained from the (same) patient at a point in time within < 30 minutes and 60 minutes after awakening (= baseline level). Said levels are then used to calculate an average value (e.g. mean, median or modal value).

In particular, the level of the at least one compound represents an average level of levels determined in a sample of exhaled breath obtained from a patient suspected of having schizophrenia about 2 minutes ($\pm$ 1 minute) after awakening, 30 minutes after awakening, and 60 minutes after awakening (= baseline level).

As mentioned above, the level is compared to a reference level. In this respect, it should be noted that the reference level of the control subject is preferably determined/calculated in the same way as the level of the patient to be tested. If, for example, an average level is determined, this level is also compared to an average reference level.

The level may also be compared to an empirically determined (median) cut-off score. Particularly, the (median) cut-off score is determined by the weighted sum of the levels of the compounds as defined above.

In a preferred embodiment, the (median) cut-off score (also designated as "*f (m060, m085, m090)* ", wherein m060 stands for trimethylamine, m085 stands for a substituted hexane, and m090 stands for an ester of butyric acid), is calculated according to the following formula:

$$f(m060, m085, m090) = \frac{1}{1 + e^{-(-6 + 0.0237*m060 + 0.3980*m085 + 0.0981*m090)}},$$

wherein

patients with f $\geq$ 0.5 are considered as healthy, i.e. as not suffering from schizophrenia and patients with f < 0.5 are considered as suffering from schizophrenia.

In a second aspect, the present invention relates to a method of determining the course of schizophrenia in a patient (having schizophrenia) comprising the step of:

determining the level of at least one compound (e.g. at least 1, 2, 3, 4, 5, 6, or 7 compound(s)) in a sample of exhaled breath obtained from a patient (having schizophrenia),
wherein the at least one compound (e.g. at least 1, 2, 3, 4, 5, 6, or 7 compound(s)) is selected from the group consisting of trimethylamine (TMA), a substituted hexane, an ester of butyric acid, acetone, isoprene, dihydropyrrole, and methylguanidine.

Preferably, the at least one compound is selected from the group consisting of trimethylamine (TMA), a substituted hexane, more preferably 1-hexanol or 2-hexanole, and an ester of butyric acid.

For example, the level of trimethylamine (TMA), the level of a substituted hexane, or the level of an ester of butyric acid is determined. For example, the level of trimethylamine (TMA) is determined and the level of a substituted hexane is determined. For example, the level of trimethylamine (TMA) is determined and the level of an ester of butyric acid is determined. For example, the level of a substituted hexane is determined and the level of an ester of butyric acid is determined. For example, the level of trimethylamine (TMA) is determined, the level of a substituted hexane is determined, and the level of an ester of butyric acid is determined. The substituted hexane is specifically 1-hexanol or 2-hexanole.

In one embodiment, the level of the at least one compound is compared to a reference level of said at least one compound. Thus, in one particular embodiment, the present invention relates to a method of determining the course of schizophrenia in a patient (having schizophrenia) which comprises the steps of:

(i) determining the level of at least one compound (e.g. at least 1, 2, 3, 4, 5, 6, or 7 compound(s)) in a sample of exhaled breath obtained from a patient (having schizophrenia), and
(ii) comparing the level of the at least one compound (e.g. at least 1, 2, 3, 4, 5, 6, or 7 compound(s)) to a reference level of said at least one compound,

wherein the at least one compound is selected from the group consisting of trimethylamine (TMA), a substituted hexane, an ester of butyric acid, acetone, isoprene, dihydropyrrole, and methylguanidine.

The above comparison allows to determine the course of schizophrenia in the patient having schizophrenia. It may be determined that schizophrenia worsens in the patient, that schizophrenia does not worsen/is stable/is not progressing in the patient, or that schizophrenia improves in the patient.

The reference level may be any level which allows to determine the course of schizophrenia in the patient. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one subject having schizophrenia, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having schizophrenia. It is more preferred that the reference level is the level determined by measuring between 2 and 500 reference samples of exhaled breath obtained from between 2 and 500 subjects having schizophrenia. It is even more preferred that the reference level is determined by measuring between 50 and 500 reference samples of exhaled breath obtained from between 50 and 500 subjects having schizophrenia. It is most preferred that the reference level is determined by measuring between 100 and 500 reference samples of exhaled breath obtained from between 100 and 500 subjects having schizophrenia.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

In a specific embodiment, the reference level represents a threshold which allows to divide/separate the patients being sick from the patients being on the way to improvement/recovery .

In one preferred embodiment, the level of the at least one compound which is

(i) below the reference level indicates that schizophrenia worsens in the patient,
(ii) comparable with the reference level indicates that schizophrenia does not worsen/is stable/non-progressing in the patient, or
(iii) above the reference level indicates that schizophrenia improves in the patient.

In particular, the level of the at least one compound is at least 0.5-fold, more preferably at least 1-fold, even more preferably at least 1.5-fold, still more preferably at least 2-fold, and most preferably at least 3-fold below/above the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

"Not worsening", "stable" or "non-progressing", as mentioned above, may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Not worsening", "stable" or "non-progressing" in this respect may also mean that the detected level variation is within the accuracy of a measurement. The accuracy of a measurement depends on the measurement method used. Preferably, the level is constant over time.

In one alternative or additional embodiment, said determining comprises determining the level of the at least one compound in a sample of exhaled breath obtained from a patient (having schizophrenia) at a first point in time and in at least one further sample of exhaled breath obtained from the (same) patient at a later point in time and comparing said levels determined at the different time points. Thus, in one particular embodiment, the present invention relates to a method of determining the course of schizophrenia in a patient (having schizophrenia) which comprises the steps of:

(i) determining the level of the at least one compound (e.g. at least 1, 2, 3, 4, 5, 6, or 7 compound(s)) in a sample of exhaled breath obtained from a patient (having schizophrenia) at a first point in time and in at least one further sample of exhaled breath obtained from the (same) patient at a later point in time, and
(ii) comparing the levels determined at the different time points,

wherein the at least one compound is selected from the group consisting of trimethylamine (TMA), a substituted hexane, an ester of butyric acid, acetone, isoprene, dihydropyrrole, and methylguanidine.

In one preferred embodiment,
the level of the at least one compound which

(i) decreases over time indicates that schizophrenia worsens in the patient,
(ii) does not change over time indicates that schizophrenia does not worsen/is stable/non-progressing in the patient, or
(iii) increases over time indicates that schizophrenia improves in the patient.

"Not worsening", "stable" or "non-progressing", as mentioned above, may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Not worsening", "stable" or "non-progressing" in this respect may also mean that the detected level variation is within the accuracy of a measurement. The accuracy of a measurement depends on the measurement method used. Preferably, the level is constant over time.

It is preferred that the time period between the first point in time and the later point(s) in time amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, or at least 2 months. For example, the patient may be routinely checked, e.g. every day, every two weeks, every month. The patient may be (re)tested at 2, 3, 4, 5, 6 7, 8, 9, or 10 time points (first point in time and further point(s) in time).

The later point in time can also be designated as second, third, fourth, fifth, or sixth point in time. In a specific example, the time period between the first point in time and the second point in time amounts to 2 weeks, the time period between the second point in time and the third point in time amounts to 2 weeks, the time period between the third and fourth point in time amounts to 1 month, and the time period between each further point in time amounts to 1 month.

It is further preferred that the first point in time is within 0 and 5 minutes after awakening (= at awakening or immediately after awakening), within < 5 minutes and 30 minutes after awakening, or within < 30 minutes and 60 minutes after awakening.

In particular, the first point in time is about 2 minutes (± 1 minute) after awakening, 30 minutes after awakening, or 60 minutes after awakening.

The patient is then retested every two weeks over a time period of between one and two months and afterwards every month (first point in time and further point(s) in time).

The individual may be (re)tested at 2, 3, 4, 5, 6 7, 8, 9, or 10 time points (first point in time and further point(s) in time).

It is more preferred that a baseline level (as a first point in time) is first determined by determining the level in a sample of exhaled breath obtained from a patient having schizophrenia within 0 and 5 minutes after awakening (= at awakening or immediately after awakening), within < 5 minutes and 30 minutes after awakening, and within < 30 minutes and 60 minutes after awakening and calculating an average level of the levels determined at the different time points.

In particular, a baseline level (as a first point in time) is first determined by determining the level in a sample of exhaled breath obtained from a patient having schizophrenia about 2 minutes (± 1 minute) after awakening, 30 minutes after awakening, and 60 minutes after awakening and calculating an average level of the levels determined at the different time points. This baseline level is then further compared with a level determined at a later point in time. For example, after the determination of the baseline level, the patient is retested every two weeks over a time period of between one and two months and afterwards every month (first point in time and further point(s) in time). The patient may be (re)tested at 2, 3, 4, 5, 6 7, 8, 9, or 10 time points (first point in time and further point(s) in time).

The later point in time can also be designated as second, third, fourth, fifth, or sixth point in time. In a specific example, a baseline level (as a first point in time), as described above, is first determined. The time period between the first point in time and the second point in time then amounts to 2 weeks, the time period between the second point in time and the third point in time amounts to 2 weeks, the time period between the third and fourth point in time amounts to 1 month, and the time period between each further point in time amounts to 1 month.

As mentioned above, the level is compared to a reference level. In this respect, it should be noted that the reference level of the control subject is preferably determined/calculated in the same way as the level of the patient to be tested. If, for example, an average level is determined, this level is also compared to average reference level.

In addition to the determination of the course schizophrenia, the treatment of this disease can be monitored. It is namely preferred that the patient receives, has received, or had received a treatment, in particular therapeutic treatment, of schizophrenia during the determination of the course of schizophrenia. The treatment of schizophrenia is preferably selected from the group consisting of the administration of a drug, psychotherapy, exercise training, cognitive training, and physical rehabilitation.

In a third aspect, the present invention relates to a method of determining whether a patient (having schizophrenia) responds to a therapeutic treatment of schizophrenia comprising the step of:

determining the level of at least one compound (e.g. at least 1, 2, 3, 4, 5, 6, or 7 compound(s)) in a sample of exhaled breath obtained from a patient (having schizophrenia),
wherein the at least one compound (e.g. at least 1, 2, 3, 4, 5, 6, or 7 compound(s)) is selected from the group consisting of trimethylamine (TMA), a substituted hexane, an ester of butyric acid, acetone, isoprene, dihydropyrrole, and methylguanidine.

Preferably, the at least one compound is selected from the group consisting of trimethylamine (TMA), a substituted hexane, more preferably 1-hexanol or 2-hexanole, and an ester of butyric acid.

For example, the level of trimethylamine (TMA), the level of a substituted hexane, or the level of an ester of butyric acid is determined. For example, the level of trimethylamine (TMA) is determined and the level of a substituted hexane is determined. For example, the level of trimethylamine (TMA) is determined and the level of an ester of butyric acid is determined. For example, the level of a substituted hexane is determined and the level of an ester of butyric acid is determined. For example, the level of trimethylamine (TMA) is determined, the level of a substituted hexane is determined, and the level of an ester of butyric acid is determined. The substituted hexane is specifically 1-hexanol or 2-hexanole.

It is preferred that the patient is a patient to whom at least once (1, 2, or 3 times) a drug to be used in said therapeutic treatment is administered, has been administered, or had been administered, and/or to whom at least once (1, 2, or 3 times) another form of therapeutic treatment (than the administration of a drug) is administered, has been administered, or had been administered. Usually, it takes at least 2 to 3 weeks before it can be determined whether the treatment is effective. During this time period, the patient has already received a therapeutic treatment 14 to 21 times. Thus, the term "at least once" covers the first treatment and any further treatment which is required.

The way of administration may be oral, nasal, rectal, parenteral, vaginal, or topical. Parental administration includes subcutaneous, intracutaneous, intramuscular, intravenous or intraperitoneal administration.

It is further preferred that the sample of exhaled breath is obtained from the patient after at least the first (e.g. first, second, or third) administration of said drug and/or after at least the first (e.g. first, second, or third) administration of another form of therapeutic treatment (than the administration of a drug).

It is particularly preferred that the sample of exhaled breath is obtained from the patient in a time period of between 3 months and 1 day after at least the first (e.g. first, second, or third) administration of said drug and/or after at least the first (e.g. first, second, or third) administration of another form of therapeutic treatment (than the administration of a drug). It is particularly more preferred that the sample of exhaled breath is obtained from the patient in a time period of between 2 month and 2 weeks after at least the first (e.g. first, second, or third) administration of said drug and/or after at least the first (e.g. first, second, or third) administration of another form of therapeutic treatment (than the administration of a drug). For example, the sample of exhaled breath is obtained from the patient 1, 2, 3, 4, 5, 6 day(s), 1, 2, 3 week(s), 1, 2, or 3 month(s) after at least the first (e.g. first, second, or third) administration of said drug and/or after at least the first (e.g. first, second, or third) administration of another form of therapeutic treatment (than the administration of a drug).

In one embodiment, the level of the at least one compound is compared to a reference level of said at least one compound. Thus, in a particular embodiment, the present invention relates to a method of determining whether a patient (having schizophrenia) responds to a therapeutic treatment of schizophrenia which comprises the steps of:

(i) determining the level of at least one compound (e.g. at least 1, 2, 3, 4, 5, 6, or 7 compound(s)) in a sample of exhaled breath obtained from a patient (having schizophrenia), and
(ii) comparing the level of the at least one compound (e.g. at least 1, 2, 3, 4, 5, 6, or 7 compound(s)) to a reference level of said at least one compound,

wherein the at least one compound is selected from the group consisting of trimethylamine (TMA), a substituted hexane, an ester of butyric acid, acetone, isoprene, dihydropyrrole, and methylguanidine.

The reference level may be any level that allows to determine whether the patient suffering from schizophrenia responds to the therapeutic treatment or not. It is preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one subject having schizophrenia, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 subject(s) having schizophrenia. It is more preferred that the reference level is the level determined by measuring between 2 and 500 reference samples of exhaled breath obtained from between 2 and 500 subjects having schizophrenia. It is even more preferred that the reference level is determined by measuring between 50 and 500 reference samples of exhaled breath obtained from between 50 and 500 subjects having schizophrenia. It is most preferred that the reference level is determined by measuring between 100 and 500 reference samples of exhaled breath obtained from between 100 and 500 subjects having schizophrenia.

It is also preferred that the reference level is the level determined by measuring at least one reference sample of exhaled breath, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 reference sample(s) of exhaled breath, obtained from at least one healthy subject, e.g. obtained from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 150, 200, 250, 300, 400, 500, or 1.000 healthy subject(s). It is more preferred that the reference level is the level determined by measuring between 2 and 500 reference samples of exhaled breath obtained from between 2 and 500 healthy subjects. It is even more preferred that the reference level is determined by measuring between 50 and 500 reference samples of exhaled breath obtained from between 50 and 500 healthy subjects. It is most preferred that the reference level is determined by measuring between 100 and 500 reference samples of exhaled breath obtained from between 100 and 500 healthy subjects.

It is practicable to take one reference sample of exhaled breath per subject for analysis. If additional reference samples of exhaled breath are required, such as to determine the reference level in different reference samples of exhaled breath (e.g. at different points in time), the same subject may be (re)tested. Said reference level may be an average reference level. It may be determined by measuring reference levels and calculating the "average" value (e.g. mean, median or modal value) thereof.

In a specific embodiment, the reference level represents a threshold which allows to divide/separate the patients in responders and non-responders.

In one alternative or additional embodiment, the reference level is the level determined in a reference sample of exhaled breath obtained from the (same) patient prior to the administration of said drug and/or prior to the administration of another form of therapeutic treatment (than the administration of a drug). It is particularly preferred that the reference sample of exhaled breath is obtained from the (same) patient in a time period of between 1 week and immediately prior to the administration of said drug and/or prior to the administration of another form of therapeutic treatment (than the administration of a drug). It is particularly more preferred that the reference sample of exhaled breath is obtained from the (same) patient in a time period of between 1 day and immediately prior to the administration of said drug and/or prior to the administration of another form of therapeutic treatment (than the administration of a drug). For example, the reference sample of exhaled breath is obtained from the (same) patient immediately, 10, 20, 30, 40, 50 minutes, 1, 2, 3, 4, 5, 6, 7, 8, 9, 10, 11, 12, 13, 14, 15, 16, 17, 18, 19, 20, 21, 22, 23 hour(s), 1, 2, 3, 4, 5, 6 days, or 1 week prior to the administration of said drug and/or prior to the administration of another form of therapeutic treatment (than the administration of a drug).

It is preferred (with respect to both embodiments described above) that the level of the at least one compound above the reference level indicates that the patient responds to said treatment of schizophrenia.

In contrast thereto, a level of the at least one compound which is comparable (e.g. identical) with or lower than the reference level indicates that the patient does not respond to said treatment of schizophrenia.

In particular, the level of the at least one compound is at least 0.5-fold, more preferably at least 1-fold, even more preferably at least 1.5-fold, still more preferably at least 2-fold, and most preferably at least 3-fold below/above the reference level. For example, the level of the at least one compound is at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

It is specifically preferred that the level/reference level is determined in a sample of exhaled breath obtained from a patient (suffering from/having schizophrenia) at a point in time within 0 and 5 minutes after awakening (= at awakening or immediately after awakening), at a point in time within < 5 minutes and 30 minutes after awakening, or at a point in time within < 30 minutes and 60 minutes after awakening. In particular, the level/reference level is determined in a sample of exhaled breath obtained from a patient (suffering from/having schizophrenia) about 2 minutes ($\pm$ 1 minute) after awakening, 30 minutes after awakening, or 60 minutes after awakening.

It is specifically more preferred that the level/reference level represents an average level of levels determined in a sample of exhaled breath obtained from a patient (suffering from/having schizophrenia) at a point in time within 0 and 5 minutes after awakening (= at awakening or immediately after awakening), at a point in time within < 5 minutes and 30 minutes after awakening, and at a point in time within < 30 minutes and 60 minutes after awakening (= baseline level). In particular, the level/reference level represents an average level of levels determined in a sample of exhaled breath obtained from a patient suffering from/having schizophrenia at awaking, 30 minutes after awakening, and 60 minutes after awakening (= baseline level).

If the treatment encompasses the administration of a drug and the patient responds to said treatment, the drug administration may be continued, the dose of the drug may be reduced, or the drug administration may be stopped. If the treatment encompasses the administration of a drug and the patient does not respond to said treatment, the dose of the drug may be increased, the drug may be changed, or the therapy mode may be changed.

It is also (additionally or alternatively) preferred that

the drug to be used in said treatment is selected from the group consisting of an antipsychotic drug, an atypical antipsychotic drug, and a typical antipsychotic drug, and/or the other form of therapeutic treatment is selected from the group consisting of psychotherapy, exercise training, cognitive remediation, cognitive training, and physical rehabilitation. Specifically, the antipsychotic drug is selected from the group consisting of clozapine, risperidone, olanzapine, aripiprazole, lumateperone, quetiapine, paliperidone, amisulpride, cariprazine, asenapine, loxapine, lurasidone, ziprasidone, sertindol, brexpiprazol, benperidol, bromperidol, chlorprotixen, flupentixol, fluphenazin, fluspirilen, haloperidol, levomepromazine, melperon, perazin, perphenazin, pimozid, pipamperone, prothipendyl, sulpiride, thioridazine, and zuclopenthixol.

The following applies to the first to third aspect of the present invention:

Diverse forms of schizophrenia are known. In the first to third aspect of the present invention, the schizophrenia may be a paranoid schizophrenia, hebephrenic schizophrenia, catatonic schizophrenia, schizophrenia simplex, or schizoaffective disorder.

The patient may be any individual suffering from schizophrenia. In the first to third aspect of the present invention, the patient is preferably a mammal, more preferably a human.

The level of the at least one compound selected from the group consisting of trimethylamine (TMA), a substituted hexane, an ester of butyric acid, acetone, isoprene, dihydropyrrole, and methylguanidine may be determined by any suitable method allowing the detection and quantification of said compound.

Preferably, the level of the at least one compound selected from the group consisting of trimethylamine (TMA), a substituted hexane, an ester of butyric acid, acetone, isoprene, dihydropyrrole, and methylguanidine is determined by spectrometry, chromatography, or a combination of both.

Suitable techniques include all mass spectrometry techniques such as time of flight mass spectrometry (TOF-MS), proton-transfer-reaction mass spectrometry (PTR-MS), quadrupole mass spectrometry (QMS), gas chromatography mass spectrometry (GC-MS), liquid chromatography mass spectrometry (LC-MS), direct infusion mass spectrometry, fourier transform ion-cyclotrone-resonance mass spectrometry (FT-ICR-MS), capillary electrophoresis mass spectrometry (CE-MS), any sequentially coupled mass spectrometry, such as MS-MS or MS-MS-MS, inductively coupled plasma mass spectrometry (ICP-MS), pyrolysis mass spectrometry (Py-MS), or ion mobility mass spectrometry.

Suitable techniques also include all chromatographic separation techniques such as liquid chromatography (LC), high performance liquid chromatography (HPLC), gas chromatography (GC), thin layer chromatography, or size exclusion or affinity chromatography. These techniques are well known in the art and can be applied by the person skilled in the art without further ado. Suitable devices for such determination are also well known in the art.

As an alternative or in addition to mass spectrometry techniques, the following techniques may be used for determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado.

More preferably

(i) the spectrometry is mass spectrometry (MS), and/or
(ii) the chromatography is gas chromatography (GC), liquid chromatography (LC) or affinity chromatography.

Even more preferably, the mass spectrometry (MS) is selected from the group consisting of Proton-Transfer-Reaction mass spectrometry (PTR-MS), Time of Flight mass spectrometry (TOF-MS), and quadrupole mass spectrometry (QMS).

All methods of the present invention may also be designated as *in vitro* methods.

In a fourth aspect, the present invention relates to the use of at least one compound (e.g. at least 1, 2, 3, 4, 5, 6, or 7 compound(s)) for diagnosing schizophrenia in a patient (suspected of having schizophrenia), for determining the course of schizophrenia in a patient (having schizophrenia), or for determining whether a patient (having schizophrenia) responds to a therapeutic treatment of schizophrenia, wherein the at least one compound (e.g. at least 1, 2, 3, 4, 5, 6, or 7 compound(s)) is selected from the group consisting of trimethylamine (TMA), a substituted hexane, an ester of butyric acid, acetone, isoprene, dihydropyrrole, and methylguanidine.

Preferably, the at least one compound is selected from the group consisting of trimethylamine (TMA), a substituted hexane, more preferably 1-hexanol or 2-hexanole, and an ester of butyric acid.

For example, the level of trimethylamine (TMA), the level of a substituted hexane, or the level of an ester of butyric acid is determined. For example, the level of trimethylamine (TMA) is determined and the level of a substituted hexane is determined. For example, the level of trimethylamine (TMA) is determined and the level of an ester of butyric acid is determined. For example, the level of a substituted hexane is determined and the level of an ester of butyric acid is determined. For example, the level of trimethylamine (TMA) is determined, the level of a substituted hexane is determined, and the level of an ester of butyric acid is determined. The substituted hexane is specifically 1-hexanol or 2-hexanole.

The diagnosing of schizophrenia allows to determine whether a patient suffers from schizophrenia or not.

The determining of the course of schizophrenia allows to determine whether schizophrenia worsens in the patient, does not worsen/is stable/not progressing in the patient, or improves in the patient.

The determining whether a patient responds to a therapeutic treatment of schizophrenia allows to examine, whether the therapeutic treatment of schizophrenia is effective or not.

In order to diagnose schizophrenia in a patient, to determine the course of schizophrenia in a patient, or to determine whether a patient responds to a therapeutic treatment of schizophrenia, the level of the at least one compound (e.g. at least 1, 2, 3, 4, 5, 6, or 7 compound(s)) selected from the group consisting of trimethylamine (TMA), a substituted hexane, an ester of butyric acid, acetone, isoprene, dihydropyrrole, and methylguanidine is determined in a sample of exhaled breath of said patient.

Diverse forms of schizophrenia are known. Preferably, the schizophrenia is selected from the group consisting of paranoid schizophrenia, hebephrenic schizophrenia, catatonic schizophrenia, and schizophrenia simplex.

The patient may be any individual suffering from schizophrenia. Preferably, the patient is a mammal, more preferably a human.

In a fifth aspect, the present invention relates to a device comprising

(i) an inlet for receiving exhaled breath, and
(ii) an analysis unit configured to analyze the exhaled breath,

wherein the analysis comprises the determination of the level of at least one compound (e.g. at least 1, 2, 3, 4, 5, 6, or 7 compound(s)) in a sample of exhaled breath obtained from a patient, and
wherein the at least one compound is selected from the group consisting of trimethylamine (TMA), a substituted hexane, an ester of butyric acid, acetone, isoprene, dihydropyrrole, and methylguanidine.

The device is capable of performing the methods of the first to third aspect.

In one preferred embodiment, the device further comprises an output unit which is configured to provide an indication to the user which is characteristic for the result of the analysis.

In one more preferred embodiment, the device is a handheld device or a portable device.

In a sixth aspect, the present invention relates to a kit comprising the device of the fifth aspect.

In one preferred embodiment, the kit comprises instructions on how to carry out the methods of the first to third aspect.

In one more preferred embodiment, the kit is useful for conducting the methods of the first to third aspect.

The invention is summarized as follows:

1. A method of diagnosing schizophrenia in a patient (suspected of having schizophrenia) comprising the step of:

> determining the level of at least one compound in a sample of exhaled breath obtained from a patient (suspected of having schizophrenia),
> wherein the at least one compound is selected from the group consisting of trimethylamine (TMA), a substituted hexane, an ester of butyric acid, acetone, isoprene, dihydropyrrole, and methylguanidine.

2. The method of item 1, wherein the at least one compound is selected from the group consisting of trimethylamine (TMA), a substituted hexane, preferably 1-hexanol or 2-hexanole, and an ester of butyric acid.

3. The method of items 1 or 2, wherein the level of the at least one compound is compared to a reference level of said at least one compound.

4. The method of item 3, wherein the reference level is the level determined by measuring at least one reference sample of exhaled breath obtained from at least one healthy subj ect.

5. The method of item 4, wherein the level of the at least one compound below the reference level indicates that the patient has schizophrenia.

6. A method of determining the course of schizophrenia in a patient (having schizophrenia) comprising the step of:

> determining the level of at least one compound in a sample of exhaled breath obtained from a patient (having schizophrenia),
> wherein the at least one compound is selected from the group consisting of trimethylamine (TMA), a substituted hexane, an ester of butyric acid, acetone, isoprene, dihydropyrrole, and methylguanidine.

7. The method of item 6, wherein the at least one compound is selected from the group consisting of trimethylamine (TMA), a substituted hexane, preferably 1-hexanol or 2-hexanole, and an ester of butyric acid.

8. The method of items 6 or 7, wherein the level of the at least one compound is compared to a reference level of said at least one compound.

9. The method of item 8, wherein the reference level is the level determined by measuring at least one reference sample of exhaled breath obtained from at least one subject having schizophrenia.

10. The method of any one of items 6 to 9, wherein said determining comprises determining the level of the at least one compound in a sample of exhaled breath obtained from a patient (having schizophrenia) at a first point in time and in at least one further sample of exhaled breath obtained from the (same) patient at a later point in time and comparing said levels determined at the different time points.

11. The method of item 10, wherein the level of the compound which

> (i) decreases over time indicates that the schizophrenia worsens in the patient,
> (ii) does not change over time indicates that the schizophrenia does not worsen/is stable in the patient, or
> (iii) increases over time indicates that the schizophrenia improves in the patient.

12. The method of any one of items 6 to 11, wherein the patient receives, has received, or had received a treatment of schizophrenia.

13. The method of item 12, wherein the treatment of schizophrenia is selected from the group consisting of the administration of a drug, psychotherapy, exercise training, cognitive training, and physical rehabilitation.

14. A method of determining whether a patient (having schizophrenia) responds to a therapeutic treatment of schizophrenia comprising the step of:

> determining the level of at least one compound in a sample of exhaled breath obtained from a patient (having schizophrenia),
> wherein the at least one compound is selected from the group consisting of trimethylamine (TMA), a substituted hexane, an ester of butyric acid, acetone, isoprene, dihydropyrrole, and methylguanidine.

15. The method of item 14, wherein the at least one compound is selected from the group consisting of trimethylamine (TMA), a substituted hexane, preferably 1-hexanol or 2-hexanole, and an ester of butyric acid.

16. The method of items 14 or 15, wherein the patient is a patient to whom at least once a drug to be used in said therapeutic treatment, and/or

> another form of therapeutic treatment
> is administered, has been administered, or had been administered.

17. The method of item 16, wherein the sample of exhaled breath is obtained from the patient after at least the first

administration of said drug and/or
administration of another form of therapeutic treatment.

18. The method of any one of items 14 to 17, wherein the level of the at least one compound is compared to a reference level of said at least one compound.

19. The method of item 18, wherein the reference level is the level determined by measuring at least one reference sample of exhaled breath obtained from at least one subject having schizophrenia.

20. The method of items 18 or 19, wherein the reference level is the level determined in a reference sample of exhaled breath obtained from the (same) patient prior to the administration of said drug, and/or administration of another form of therapeutic treatment.

21. The method of any one of items 18 to 20, wherein the level of the at least one compound above the reference level indicates that the patient responds to said treatment of schizophrenia.

22. The method of any one of items 16 to 21, wherein

the drug to be used in said treatment is selected from the group consisting of an antipsychotic drug, an atypical antipsychotic drug, and a typical antipsychotic drug, and/or
the other form of therapeutic treatment is selected from the group consisting of psychotherapy, exercise training, cognitive remediation, cognitive training, and physical rehabilitation.

23. The method of item 22, wherein the antipsychotic drug is selected from the group consisting of clozapine, risperidone, olanzapine, aripiprazole, lumateperone, quetiapine, paliperidone, amisulpride, cariprazine, asenapine, loxapine, lurasidone, ziprasidone, sertindol, brexpiprazol, benperidol, bromperidol, chlorprotixen, flupentixol, fluphenazin, fluspirilen, haloperidol, levomepromazine, melperon, perazin, perphenazin, pimozid, pipamperone, prothipendyl, sulpiride, thioridazine, and zuclopenthixol.

24. The method of any one of items 1 to 23, wherein the schizophrenia is selected from the group consisting of paranoid schizophrenia, hebephrenic schizophrenia, catatonic schizophrenia, and schizophrenia simplex.

25. The method of any one of items 1 to 24, wherein the patient is a mammal, preferably a human.

26. The method of any one of items 1 to 25, wherein the level of the at least one compound is determined by spectrometry, chromatography, or a combination of both.

27. The method of item 26, wherein

(i) the spectrometry is mass spectrometry (MS), and/or
(ii) the chromatography is gas chromatography (GC), liquid chromatography (LC) or affinity chromatography.

28. The method of item 27, wherein the mass spectrometry (MS) is selected from the group consisting of Proton-Transfer-Reaction mass spectrometry (PTR-MS), Time of Flight mass spectrometry (TOF-MS), and quadrupole mass spectrometry (QMS).

29. Use of at least one compound for diagnosing schizophrenia in a patient (suspected of having schizophrenia), for determining the course of schizophrenia in a patient (having schizophrenia), or for determining whether a patient (having schizophrenia) responds to a therapeutic treatment of schizophrenia, wherein the at least one compound is selected from the group consisting of trimethylamine (TMA), a substituted hexane, an ester of butyric acid, acetone, isoprene, dihydropyrrole, and methylguanidine.

30. The use of item 29, wherein the at least one compound is selected from the group consisting of trimethylamine (TMA), a substituted hexane, preferably 1-hexanol or 2-hexanole, and an ester of butyric acid.

31. A device comprising

(i) an inlet for receiving exhaled breath, and
(ii) an analysis unit configured to analyze the exhaled breath,

wherein the analysis comprises the determination of the level of at least one compound in a sample of exhaled breath obtained from a patient,
wherein the at least one compound is selected from the group consisting of trimethylamine (TMA), a substituted hexane, an ester of butyric acid, acetone, isoprene, dihydropyrrole, and methylguanidine.

32. The device of item 31, wherein the device further comprises an output unit which is configured to provide an indication to the user which is characteristic for the result of the analysis.

33. The device of items 31 or 32, wherein the device is a handheld device or portable device.

34. The device of any one of items 31 to 33, wherein the device is capable of performing the methods of any one of items 1 to 28.

35. A kit comprising the device of any one of items 31 to 34.

36. The kit of item 35, wherein the kit comprises instructions on how to carry out the methods of any one of items 1 to 28.

37. The kit of items 35 or 36, wherein the kit is useful for conducting the methods of any one of items 1 to 28.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

BRIEF DESCRIPTION OF THE FIGURES

The following figures and examples are merely illustrative of the present invention and should not be construed to limit the scope of the invention as indicated by the appended claims in any way.

**Figure 1:** Demographic and clinical data for patients with schizophrenia and healthy controls. Indicated are the test used (T: "Student's t-test", $X^2$: Chi-Squared test), test values, p-values, effect size test as well the effect size. Olanzapine equivalents are calculated based on Gardener et al. (International consensus study of antipsychotic dosing. Am J Psychiatry. 2010; 167:686-693).

**Figure 2:** Results of the bootstrap mixed model analysis. Shown are significant effects surviving FDR correction for multiple comparisons with N=1000 permutations.

**Figure 3:** Stability of marker compositions in the breathing air at three different times (after awakening, after 30 minutes, and after 60 minutes) for the selected best masses found by mixed model analysis

**Figure 4:** ROC curve of composite models. Depicted is sensitivity and 1-specificity for m/z 60 and m/z 60+90, m/z 60+90+85 and the BART analysis model for these markers. In the legend AUC and accuracy is given for each model.

**Figure 5:** Classification result of the linear approach by using logit regression. Shown are predicted values for patients and controls and the correct or incorrect classification.

EXAMPLES

The examples given below are for illustrative purposes only and do not limit the invention described above in any way.

1. Methods

1.1 Participants

For this study, 28 patients with a DSM-V diagnosis of schizophrenia where recruited from the psychiatric hospital Magdeburg. All patients were admitted in the hospital because of a non-drug-associated acute first-episode psychosis or re-exacerbation of psychosis and were measured after stabilization due to the conditions of consent and compliance. Accordingly, 29 healthy controls were recruited.

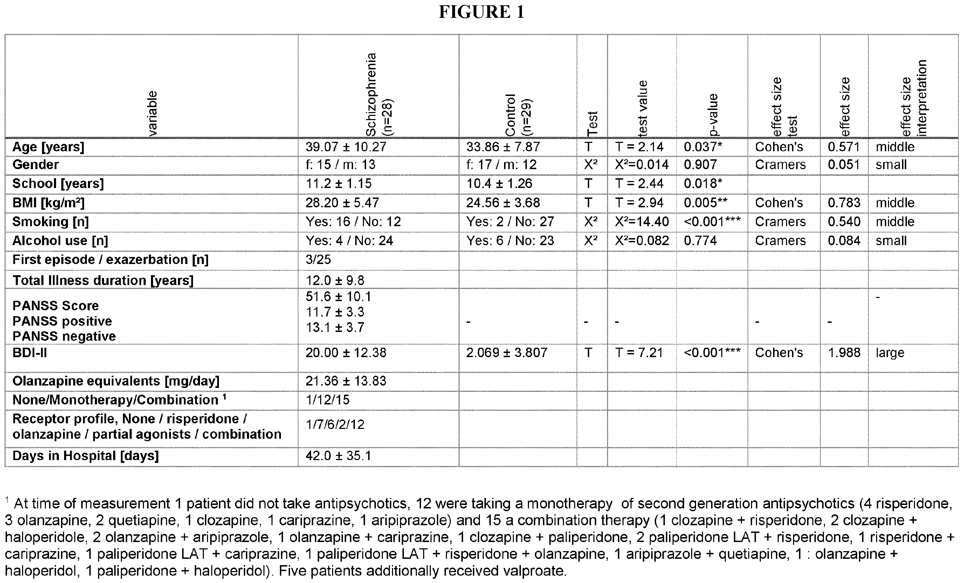

Sample characteristics are given in **FIGURE 1.** Excluded from the study were people who would not or were not able to give their consent, as well as people with a history of neurological, severe internal or hormonal disorders. Patients with acute suicidal tendencies and psychiatric comorbidities were excluded. Concerning the control group a psychiatric disorder was another exclusion criteria.

The study was approved by the local ethics committee and carried out according to declaration of Helsinki. All subjects were given a written form, detailing the process of the study, as well as oral explanations.

1.2 Clinical assessment

All subjects were clinically assessed to ensure the diagnosis of schizophrenia according to DSM-IV by professional and independent clinicians. Additionally, psychiatric interviews with each subject were performed to explore the current psychopathological state. To capture the positive and negative symptoms in schizophrenia, the "Positive and Negative Syndrome Scale (PANSS)" was used. Moreover, "Hamilton Depression Scale (HDRS)", the "Beck Depression Inventory (BDI)", and "Clinical Global Impression (CGI)", were carried out. Furthermore, participants filled out the personality questionnaires NEO-FFI and SCID-II. Side effects of medication, sleep quality, anxiety, social and employment status, early life adversity, perceived stress and life events were further assessed (Data not shown).

In addition, the clinical record with interviews and documentation were noted as well as the history of the therapy. The past-history and current application of medication and psychotherapy were also recorded. Since there is certain impact on the exhaled VOCs of people who smoke compared to non-smokers, the participants were asked about their smoking behavior. To minimize the direct impact of nutrition on the breath gas analysis, although VOCs seem to change rather later after 4 hours of food intake than immediately, the subjects carried out the breathing maneuver on an empty stomach.

## 1.3 Breath gas analysis

The breath gas analysis occurred through measuring the VOC concentrations in the breath gas of the participants via PTR-MS. Breath probes were taken from the subjects thrice, directly after awakening in the morning, after 30, and after 60 minutes, since awakening is a dynamic change from rest to activity. Thus, this period is chosen to investigate stability over several time points and secondly to investigate physiological dynamic changes in accordance to the cortisol awakening response, which was found to be blunted in patients with schizophrenia. Participants used three Tedlar bags with special tubes to collect the breathing air. Tedlar bags can also be used at home and thus allowed measurement directly after awakening in all participants. During the hour after awakening, subjects were asked not to drink anything besides clear water, not to smoke, eat or brush their teeth. The participants stayed inside and refrained from any physical activity.

Since breath gas analysis of at least some VOCs, like isoprene, is dependent on the respiratory physiology, a standard procedure was used (see Sukul P, Schubert JK, Zanaty K, Trefz P, Sinha A, Kamysek S, Miekisch W. Exhaled breath compositions under varying respiratory rhythms reflects ventilatory variations: translating breathomics towards respiratory medicine. Sci Rep. 2020; 10:14109). By the right time, all subjects sat quiet for two minutes. Then, after two minutes of paced breath rhythm, they switched to spontaneous rhythm and started breath sampling. The participants took one deep breath, opened the bag's tube with their mouth by pushing it in, exhaled deeply into the bag to fill it up, and by using the teeth, locked the bag up again. This maneuver was repeated to the times named above.

The samples were collected afterwards and measurement via the PTR-MS occurred in approximate time. The samples did not need further preparations and could be directly processed. Upon collection, the breath samples were analysed within 5 hours. VOCs can be preserved relatively stable in Tedlar bags for the first 10 hours. The PTR-MS measures the VOCs by taking in the breath gas through a drift tube and creating a reaction with protons, most commonly $H3O^+$. Masses between m/z 19 to 200 can be analyzed by the quadrupole based analyzer unit (see Blake RS, Whyte C, Hughes CO, Ellis AM, Monks PS. Demonstration of proton-transfer reaction time-of-flight mass spectrometry for real-time analysis of trace volatile organic compounds. Anal Chem. 2004;76:3841-3845). In order to better classify masses, parallel investigations with PTR-MS and PTR-TOF-MS were made, since PTR-TOF-MS allows a 100 fold more exact detection of masses with up to 2 decimal places.

## 1.4 Data analysis

To detected differences in mass concentrations between groups and over time at awakening and to account for multiple testing, bootstrap mixed model analyses (N=1000) were performed. The bootstrap p-values were adjusted by false discovery rate (FDR) method. Masses were included as dependent variables in separate models. In all models, the between-subjects factors diagnosis (factor: 1 = patients, 0 = HC), sex (factor: 0 = males, 1 = females), and the within-subject factor time (Baseline, 30 minutes, 60 minutes) were included. Age, smoking history, and BMI were used as between-subject covariates. Moreover, the interactive effect between diagnosis and time was included as well. All masses that showed a significant effect with smoking were not used in further data analysis. The sample were also tested for differences between smokers and non-smokers. The molecules that differed in these results, and had a proven link to smoking (m/z 28, 42, 79, 93, 97, 123), were excluded in further data analysis and logistic regression.

Masses showing significant effects of smoking were excluded. Furthermore, it was tested for an effect of medication by examining Pearson correlations between concentration of masses and olanzapine equivalent doses. Not adjusted p-value were taken to be more sensitive for these effects. To get a very slim classification models, logistic regression with conditional forward method of SPSS was used to select the most useful masses to classify the diagnostic groups. The model starts with the first mass, which can best classify the diagnosis groups and then the next mass with the second best classify of group are taken in the model. The process is canceled, if new added masses to the model do not improve the model significantly. After detection the most important masses, a bootstrap analysis of logistic regression of the final model with 1000 samples are used. This is done to show that the final model is robust.

The receiver operating characteristic (ROC) curves of each step of the logistic regression analysis were created to evaluate the models und show the improvements. A formula for classification was build.

Hence this linear approach is limited, a nonlinear machine learning approach was taken by using the BART machine. The BART machine is a sophisticate algorithm (see Kapelner A, Bleich, J. bartMachine: Machine Learning with Bayesian Additive Regression Trees. Journal of Statistical Software. 2016;70).

Cross validation was carried out by dividing the dataset in different sub samples. Hence the BART model is a kind of black box, no formula could be shown in order to classify the groups.

## 2. Results

### 2.1 Candidate Selection and evaluation

In terms of gender and BMI the patient group and the healthy control group had no significant differences. As for the age aspect, the control group was on average about 4 years younger than the patient group. Per definition, patients with schizophrenia suffered from positive and negative symptoms associated with the disorder compared to the healthy group (see **FIGURE 1**). They were recruited after determining that their current psychiatric status was stable enough and entered to not suffer any consequences by participating in the study.

### 2.2 Univariate Analysis

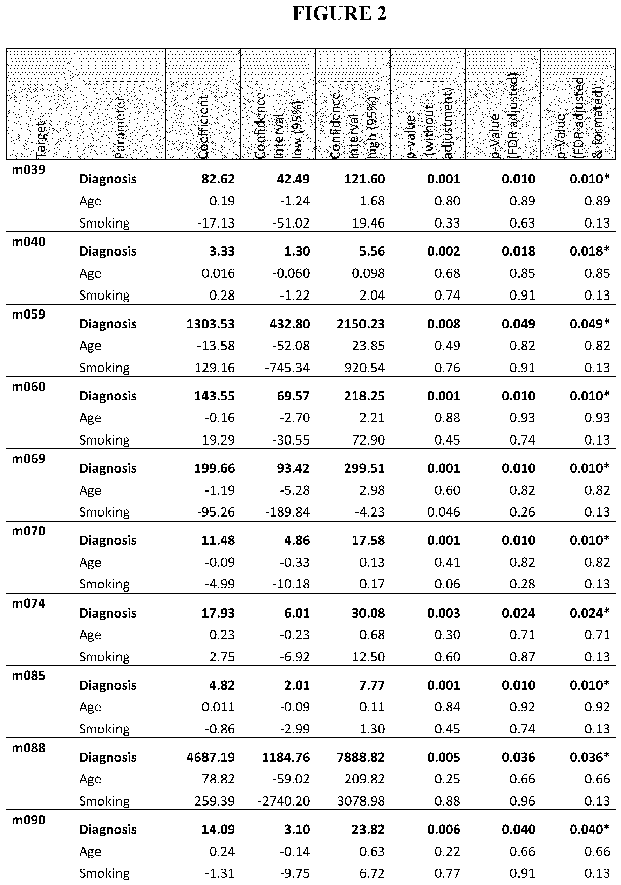

Bootstrap Mixed model analyses showed that 10 markers (m/z 39, 40, **59, 60, 69, 70, 74, 85,** 88, **90**) were significantly different in concentration between patients with schizophrenia and the healthy controls, even corrected for multiple comparisons **(FIGURE 2).**

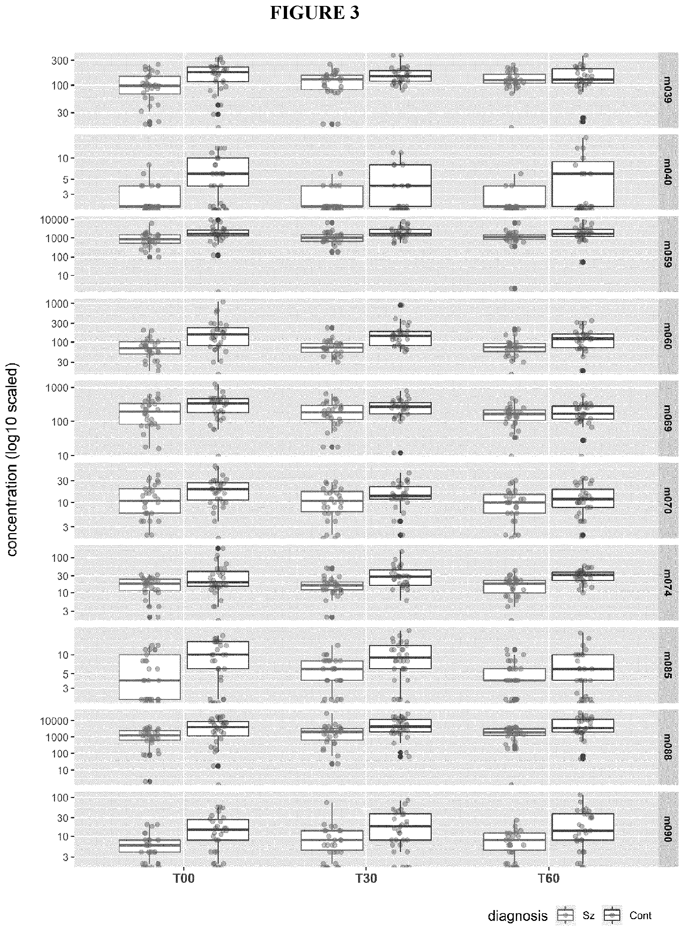

It is also noted that the time of measurement, age, and smoking had no significant impact on the concentration of the signatures shown in **FIGURE 3.**

### 2.3 Composite of marker by logistic regression models

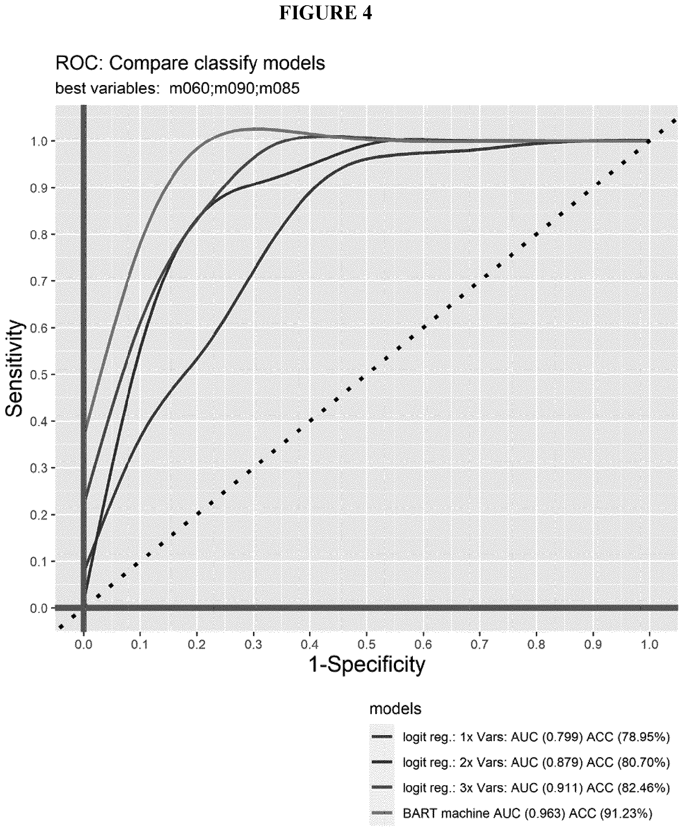

10 markers (m/z 39, 40, **59, 60, 69, 70, 74, 85,** 88, **90)** showing significant diagnoses effects in the bootstrap mixed model analysis without showing an effect of smoking were furthermore subjected to logistic regression analysis. With logistic regression by using the forward conditional method 3 masses (m/z **60, 85, 90)** were identified. Each mass significantly improved the composite logistic regression model. The stepwise improvement of the composite model is shown in **FIGURE 4.**

Using bootstrapping with this final composite model, we could show, the all 3 masses have a robust significant impact to classify the diagnosis groups. The final composite model reached an AUC of 0.91 and an accuracy of 82.4%.

The following composite formula was obtained for masses m/z **60, 85** and **90:**

$$f(m060, m085, m090) = \frac{1}{1 + e^{-(-6 + 0.0237 * m060 + 0.3980 * m085 + 0.0981 * m090)}}$$

wherein patients with f ≥ 0.5 are considered as healthy, i.e. as not suffering from schizophrenia and patients with f < 0.5 are considered as suffering from schizophrenia.

### 2.4 BART Machine Analysis

To demonstrate that using more sophisticated non-linear methods also gives better classification results, the BART machine of the 4 masses (m/z **60, 85, 90)** was applied. The classify result achieved an AUC of 0.96 and an accuracy of 91.2%.

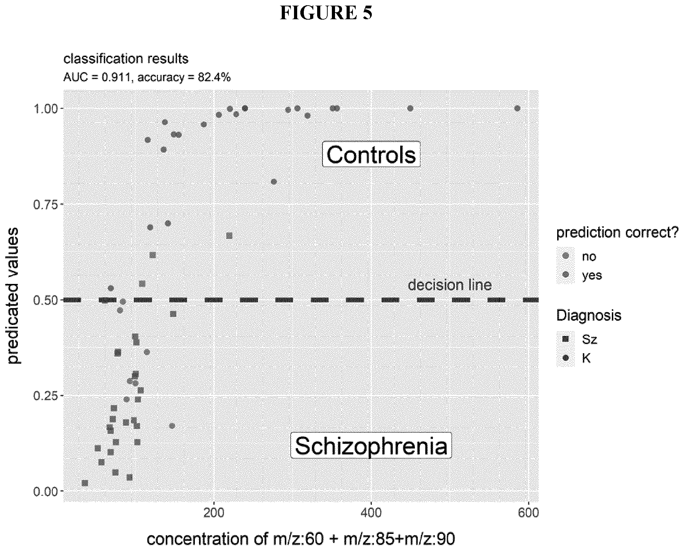

The result is also shown in **FIGURE 5.**

### 2.5. Marker identification

The following markers could finally be identified:

| m/z | identified compound |
|-----|---------------------|
| 59  | Acetone             |

(continued)

| 60 | trimethylamine (TMA) |
|---|---|
| 69 | Isoprene |
| 70 | Dihydropyrrole |
| 74 | Methylguanidine |
| 85 | substituted hexane such as 1-hexanol |
| 90 | ester of butyric acid |

3. Summary

This is a first study to investigate breath gas analysis in schizophrenia. So far, it was well tolerated and has shown good potential to differentiate between patients with schizophrenia and healthy controls. Currently, the diagnosis of schizophrenia is made by clinical assessment by experts in psychiatry. In order to support diagnostics and reduce the time until on sufficient treatment objectively measurable markers are highly warranted.

Breath gas analysis has proven to possess certain advantages such as being non-invasive and fast to apply over other biomarkers currently studied. Moreover, direct mass spectrometric techniques, such as PTR-MS that was used here, represent an innovative, quick, and non-labour-intensive approach to measure VOCs compared to other MS methods.

The patients that have participated in this study were all capable of executing the breath gas sampling by themselves after the steps were explained to them. Patients with schizophrenia displayed significantly lower levels for markers m/z 39, 40, 59, 60, 69, 70, 74, 85, 88, 90 compared to the healthy control group. None of these were associated with smoking, medication, days in hospital or illness duration. Following the logistic regression analysis with bootstrapping, the markers m/z **60, 85** and **90** were consequently used to create a formula for predicting diagnosis.

**Claims**

**1.** A method of diagnosing schizophrenia in a patient (suspected of having schizophrenia) comprising the step of:

determining the level of at least one compound in a sample of exhaled breath obtained from a patient (suspected of having schizophrenia),
wherein the at least one compound is selected from the group consisting of trimethylamine (TMA), a substituted hexane, an ester of butyric acid, acetone, isoprene, dihydropyrrole, and methylguanidine.

**2.** The method of claim 1,

wherein the level of the at least one compound is compared to a reference level of said at least one compound,
wherein preferably the reference level is the level determined by measuring at least one reference sample of exhaled breath obtained from at least one healthy subject,
wherein more preferably the level of the at least one compound below the reference level indicates that the patient has schizophrenia.

**3.** A method of determining the course of schizophrenia in a patient (having schizophrenia) comprising the step of:

determining the level of at least one compound in a sample of exhaled breath obtained from a patient (having schizophrenia),
wherein the at least one compound is selected from the group consisting of trimethylamine (TMA), a substituted hexane, an ester of butyric acid, acetone, isoprene, dihydropyrrole, and methylguanidine.

**4.** The method of claim 3,

wherein said determining comprises determining the level of the at least one compound in a sample of exhaled breath obtained from a patient (having schizophrenia) at a first point in time and in at least one further sample of exhaled breath obtained from the (same) patient at a later point in time and comparing said levels determined at the different time points,
wherein preferably the level of the compound which

    (i) decreases over time indicates that the schizophrenia worsens in the patient,
    (ii) does not change over time indicates that the schizophrenia does not worsen/is stable in the patient, or
    (iii) increases over time indicates that the schizophrenia improves in the patient.

5. The method of claims 3 or 4,
wherein the patient receives, has received, or had received a treatment of schizophrenia, wherein preferably the treatment of schizophrenia is selected from the group consisting of the administration of a drug, psychotherapy, exercise training, cognitive training, and physical rehabilitation.

6. A method of determining whether a patient (having schizophrenia) responds to a therapeutic treatment of schizophrenia comprising the step of:

determining the level of at least one compound in a sample of exhaled breath obtained from a patient (having schizophrenia),
wherein the at least one compound is selected from the group consisting of trimethylamine (TMA), a substituted hexane, an ester of butyric acid, acetone, isoprene, dihydropyrrole, and methylguanidine.

7. The method of claim 6, wherein the patient is a patient to whom at least once a drug to be used in said therapeutic treatment, and/or

another form of therapeutic treatment
is administered, has been administered, or had been administered.

8. The method of claim 7, wherein the sample of exhaled breath is obtained from the patient after at least the first

administration of said drug and/or
administration of another form of therapeutic treatment.

9. The method of any one of claims 6 to 8, wherein the level of the at least one compound is compared to a reference level of said at least one compound.

10. The method of claim 9, wherein the reference level is the level determined by measuring at least one reference sample of exhaled breath obtained from at least one subject having schizophrenia.

11. The method of claims 9 or 10, wherein the reference level is the level determined in a reference sample of exhaled breath obtained from the (same) patient prior to the administration of said drug, and/or
administration of another form of therapeutic treatment.

12. The method of any one of claims 9 to 11, wherein the level of the at least one compound above the reference level indicates that the patient responds to said treatment of schizophrenia.

13. Use of at least one compound for diagnosing schizophrenia in a patient (suspected of having schizophrenia), for determining the course of schizophrenia in a patient (having schizophrenia), or for determining whether a patient (having schizophrenia) responds to a therapeutic treatment of schizophrenia, wherein the at least one compound is selected from the group consisting of trimethylamine (TMA), a substituted hexane, an ester of butyric acid, acetone, isoprene, dihydropyrrole, and methylguanidine.

14. A device comprising

(i) an inlet for receiving exhaled breath, and
(ii) an analysis unit configured to analyze the exhaled breath,
wherein the analysis comprises the determination of the level of at least one compound in a sample of exhaled breath obtained from a patient,
wherein the at least one compound is selected from the group consisting of trimethylamine (TMA), a substituted hexane, an ester of butyric acid, acetone, isoprene, dihydropyrrole, and methylguanidine.

**15.** A kit comprising the device of claim 14.

## FIGURE 1

| Variable | Schizophrenia (n=28) | Control (n=29) | Test | test value | p-value | effect size test | effect size | interpretation effect size |
|---|---|---|---|---|---|---|---|---|
| Age [years] | 39.07 ± 10.27 | 33.86 ± 7.87 | T | T = 2.14 | 0.037* | Cohen's | 0.571 | middle |
| Gender | f: 15 / m: 13 | f: 17 / m: 12 | X² | X²=0.014 | 0.907 | Cramers | 0.051 | small |
| School [years] | 11.2 ± 1.15 | 10.4 ± 1.26 | T | T = 2.44 | 0.018* | Cohen's | 0.783 | middle |
| BMI [kg/m²] | 28.20 ± 5.47 | 24.56 ± 3.68 | T | T = 2.94 | 0.005** | Cohen's | 0.540 | middle |
| Smoking [n] | Yes: 16 / No: 12 | Yes: 2 / No: 27 | X² | X²=14.40 | <0.001*** | Cramers | | |
| Alcohol use [n] | Yes: 4 / No: 24 | Yes: 6 / No: 23 | X² | X²=0.082 | 0.774 | Cramers | 0.084 | small |
| First episode / exazerbation [n] | 3/25 | | | | | | | |
| Total Illness duration [years] | 12.0 ± 9.8 | | | | | | | |
| PANSS Score PANSS positive PANSS negative | 51.6 ± 10.1 11.7 ± 3.3 13.1 ± 3.7 | - | - | - | | - | - | - |
| BDI-II | 20.00 ± 12.38 | 2.069 ± 3.807 | T | T = 7.21 | <0.001*** | Cohen's | 1.988 | large |
| Olanzapine equivalents [mg/day] | 21.36 ± 13.83 | | | | | | | |
| None/Monotherapy/Combination [1] | 1/12/15 | | | | | | | |
| Receptor profile, None / risperidone / olanzapine / partial agonists / combination | 1/7/6/2/12 | | | | | | | |
| Days in Hospital [days] | 42.0 ± 35.1 | | | | | | | |

[1] At time of measurement 1 patient did not take antipsychotics, 12 were taking a monotherapy of second generation antipsychotics (4 risperidone, 3 olanzapine, 2 quetiapine, 1 clozapine, 1 cariprazine, 1 aripiprazole) and 15 a combination therapy (1 clozapine + risperidone, 2 clozapine + haloperidole, 2 olanzapine + aripiprazole, 1 olanzapine + cariprazine, 1 clozapine + paliperidone, 2 paliperidone LAT + risperidone, 1 risperidone + cariprazine, 1 paliperidone LAT + cariprazine, 1 paliperidone LAT + risperidone + olanzapine, 1 aripiprazole + quetiapine, 1 : olanzapine + haloperidol, 1 paliperidone + haloperidol). Five patients additionally received valproate.

# FIGURE 2

| Target | Parameter | Coefficient | Confidence Interval low (95%) | Confidence Interval high (95%) | p-value (without adjustment) | p-Value (FDR adjusted) | p-Value (FDR adjusted & formated) |
|---|---|---|---|---|---|---|---|
| m039 | Diagnosis | 82.62 | 42.49 | 121.60 | 0.001 | 0.010 | 0.010* |
| | Age | 0.19 | -1.24 | 1.68 | 0.80 | 0.89 | 0.89 |
| | Smoking | -17.13 | -51.02 | 19.46 | 0.33 | 0.63 | 0.13 |
| m040 | Diagnosis | 3.33 | 1.30 | 5.56 | 0.002 | 0.018 | 0.018* |
| | Age | 0.016 | -0.060 | 0.098 | 0.68 | 0.85 | 0.85 |
| | Smoking | 0.28 | -1.22 | 2.04 | 0.74 | 0.91 | 0.13 |
| m059 | Diagnosis | 1303.53 | 432.80 | 2150.23 | 0.008 | 0.049 | 0.049* |
| | Age | -13.58 | -52.08 | 23.85 | 0.49 | 0.82 | 0.82 |
| | Smoking | 129.16 | -745.34 | 920.54 | 0.76 | 0.91 | 0.13 |
| m060 | Diagnosis | 143.55 | 69.57 | 218.25 | 0.001 | 0.010 | 0.010* |
| | Age | -0.16 | -2.70 | 2.21 | 0.88 | 0.93 | 0.93 |
| | Smoking | 19.29 | -30.55 | 72.90 | 0.45 | 0.74 | 0.13 |
| m069 | Diagnosis | 199.66 | 93.42 | 299.51 | 0.001 | 0.010 | 0.010* |
| | Age | -1.19 | -5.28 | 2.98 | 0.60 | 0.82 | 0.82 |
| | Smoking | -95.26 | -189.84 | -4.23 | 0.046 | 0.26 | 0.13 |
| m070 | Diagnosis | 11.48 | 4.86 | 17.58 | 0.001 | 0.010 | 0.010* |
| | Age | -0.09 | -0.33 | 0.13 | 0.41 | 0.82 | 0.82 |
| | Smoking | -4.99 | -10.18 | 0.17 | 0.06 | 0.28 | 0.13 |
| m074 | Diagnosis | 17.93 | 6.01 | 30.08 | 0.003 | 0.024 | 0.024* |
| | Age | 0.23 | -0.23 | 0.68 | 0.30 | 0.71 | 0.71 |
| | Smoking | 2.75 | -6.92 | 12.50 | 0.60 | 0.87 | 0.13 |
| m085 | Diagnosis | 4.82 | 2.01 | 7.77 | 0.001 | 0.010 | 0.010* |
| | Age | 0.011 | -0.09 | 0.11 | 0.84 | 0.92 | 0.92 |
| | Smoking | -0.86 | -2.99 | 1.30 | 0.45 | 0.74 | 0.13 |
| m088 | Diagnosis | 4687.19 | 1184.76 | 7888.82 | 0.005 | 0.036 | 0.036* |
| | Age | 78.82 | -59.02 | 209.82 | 0.25 | 0.66 | 0.66 |
| | Smoking | 259.39 | -2740.20 | 3078.98 | 0.88 | 0.96 | 0.13 |
| m090 | Diagnosis | 14.09 | 3.10 | 23.82 | 0.006 | 0.040 | 0.040* |
| | Age | 0.24 | -0.14 | 0.63 | 0.22 | 0.66 | 0.66 |
| | Smoking | -1.31 | -9.75 | 6.72 | 0.77 | 0.91 | 0.13 |

# FIGURE 3

## FIGURE 4

ROC: Compare classify models
best variables:  m060;m090;m085

models

— logit reg.: 1x Vars: AUC (0.799) ACC (78.95%)

— logit reg.: 2x Vars: AUC (0.879) ACC (80.70%)

— logit reg.: 3x Vars: AUC (0.911) ACC (82.46%)

— BART machine AUC (0.963) ACC (91.23%)

**FIGURE 5**

classification results
AUC = 0.911, accuracy = 82.4%

Europäisches
Patentamt

European
Patent Office

Office européen
des brevets

# EUROPEAN SEARCH REPORT

Application Number

EP 22 15 6860

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/344281 A1 (AHMAD LUBNA M [US] ET AL) 14 November 2019 (2019-11-14) | 14,15 | INV. A61B5/00 |
| A | * paragraphs [0080] – [0108]; claims 1,5; figures 1,26 * | 1-13 | ADD. A61B5/08 |
| X | US 2018/038825 A1 (RATTO TIMOTHY [US] ET AL) 8 February 2018 (2018-02-08) * the whole document * | 14,15 | |
| X | US 2019/313944 A1 (SUN NIAN XIANG [US] ET AL) 17 October 2019 (2019-10-17) * paragraphs [0082] – [0085] * | 14,15 | |
| A | PHILLIPS M ET AL: "Volatile organic compounds in the breath of patients with schizophrenia.", JOURNAL OF CLINICAL PATHOLOGY, vol. 48, no. 5, 1 May 1995 (1995-05-01), pages 466-469, XP055944827, GB ISSN: 0021-9746, DOI: 10.1136/jcp.48.5.466 Retrieved from the Internet: URL:https://www.ncbi.nlm.nih.gov/pmc/articles/PMC502626/pdf/jclinpath00230-0076.pdf> * the whole document * | 1,3,6,13 | TECHNICAL FIELDS SEARCHED (IPC) A61B |
| A | GIANNOUKOS S ET AL: "Volatolomics: A broad area of experimentation", JOURNAL OF CHROMATOGRAPHY B, vol. 1105, 15 January 2019 (2019-01-15), pages 136-147, XP085574303, ISSN: 1570-0232, DOI: 10.1016/J.JCHROMB.2018.12.015 * table 2 * | 1,3,6,13 | |

The present search report has been drawn up for all claims

| Place of search | Date of completion of the search | Examiner |
|---|---|---|
| Berlin | 21 July 2022 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 6860

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2022

| Patent document cited in search report | | Publication date | Patent family member(s) | | Publication date |
|---|---|---|---|---|---|
| US 2019344281 | A1 | 14-11-2019 | US | 9643186 B1 | 09-05-2017 |
| | | | US | 2018056302 A1 | 01-03-2018 |
| | | | US | 2019344281 A1 | 14-11-2019 |
| US 2018038825 | A1 | 08-02-2018 | NONE | | |
| US 2019313944 | A1 | 17-10-2019 | US | 2019313944 A1 | 17-10-2019 |
| | | | US | 2022167873 A1 | 02-06-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- A multilingual glossary of biotechnological terms: (IUPAC Recommendations). Helvetica Chimica Acta. 1995
- **GARDENER et al.** International consensus study of antipsychotic dosing. *Am J Psychiatry,* 2010, vol. 167, 686-693
- **SUKUL P ; SCHUBERT JK ; ZANATY K ; TREFZ P ; SINHA A ; KAMYSEK S ; MIEKISCH W.** Exhaled breath compositions under varying respiratory rhythms reflects ventilatory variations: translating breathomics towards respiratory medicine. *Sci Rep.,* 2020, vol. 10, 14109
- **BLAKE RS ; WHYTE C ; HUGHES CO ; ELLIS AM ; MONKS PS.** Demonstration of proton-transfer reaction time-of-flight mass spectrometry for real-time analysis of trace volatile organic compounds. *Anal Chem.,* 2004, vol. 76, 3841-3845
- **KAPELNER A ; BLEICH, J.** bartMachine: Machine Learning with Bayesian Additive Regression Trees. *Journal of Statistical Software,* 2016, vol. 70